# EUROPEAN PATENT SPECIFICATION

(11) **EP 3 934 759 B1**
(45) Date of publication and mention of the grant of the patent: **29.07.2026**
(21) Application number: 20770763.9
(22) Date of filing: 06.03.2020
(51) Int. Cl.: A61K 31/7052, A61K 31/195, A61P 31/12, A61K 31/522

(54) **FILOCICLOVIR FOR THE TREATMENT OF ADENOVIRUS INFECTION**
FILIOCICLOVIR ZUR BEHANDLUNG VON ADENOVIRUS-INFEKTIONEN
FILOCICLOVIR POUR LE TRAITEMENT D'UNE INFECTION PAR UN ADÉNOVIRUS

(30) Priority: 08.03.2019 US 201962815630 P
(43) Date of publication of application: 12.01.2022
(73) Proprietor: Microbiotix, Inc., Worcester, MA 01605 (US); The UAB Research Foundation, Birmingham AL 35233 (US)
(72) Inventor: BOWLIN, Terry, L., Maineville, OH 45039 (US); PRICHARD, Mark, N., deceased (US)
(74) Representative: Braeuning Schubert Patentanwälte GbR
(86) International application number: PCT/US2020/021548
(87) International publication number: WO 2020/185620

(56) References cited:
- US-A1- 2007 123 566
- CAROLL B. HARTLINE, KEITH KATHY A., EAGAR JESSICA, HARDEN EMMA A., BOWLIN TERRY L., PRICHARD MARK N.: "A standardized approach to the evaluation of antivirals against DNA viruses: Orthopox-, adeno-, and herpesviruses", ANTIVIRAL RESEARCH, vol. 159, 1 October 2018 (2018-10-01) - 1 November 2018 (2018-11-01), pages 104 - 112, XP055739179
- ANONYMOUS: "Safety and PK of MBX-400 (Cyclopropavir) in Normal Volunteers", CLINICALTRIALS.GOV, 26 December 2017 (2017-12-26), pages 2, XP055739183, Retrieved from the Internet <URL:https://clinicaltrials.gov/ct2/show/NCT02454699> [retrieved on 20200415]
- ROY F. CHEMALY, HILL JOSHUA A., VOIGT SEBASTIAN, PEGGS KARL S.: "In vitro comparison of currently available and investigational antiviral agents against pathogenic human double-stranded DNA viruses: A systematic literature review", ANTIVIRAL RESEARCH, vol. 163, 21 January 2019 (2019-01-21) - March 2019 (2019-03-01), pages 50 - 58, XP055739189
- KEITH, K ET AL.: "A standardized approach to the evaluation of antivirals against DNA viruses: Polyomaviruses and lymphotropic herpesviruses", ANTIVIRAL RESEARCH, vol. 159, 1 October 2018 (2018-10-01) - November 2018 (2018-11-01), pages 122 - 129, XP055739198

## Description

### Field of the Invention

This invention is in the field of therapeutic and prophylactic drugs to treat adenoviral infections and disease. In particular, the present invention is directed to filociclovir (FCV) or a pharmaceutically acceptable salt thereof for use in a method of treating adenoviral infections in mammals, and in particular of treating adenoviral infections in humans.

### Background of the Invention

Adenoviruses (AdVs) are ubiquitous DNA viruses that are most commonly associated with pediatric illnesses of the respiratory tract, gastrointestinal tract and conjunctiva. Other rare manifestations of AdV infections include hepatitis, as well as genitourinary and neurologic symptoms (Khanal et al., Biomedicines, 6(1) (2018)). Although most adenoviral infections are self-limiting in the immunocompetent host, disseminated forms of the disease and subsequent high fatalities can occur in immunocompromised patients and other vulnerable populations in closed or crowded settings, such as school children and military recruits (Hendrix et al., Emerg. Infect. Dis., 5(6): 798-801 (1999); Munoz et al., Clin. Infect. Dis. 27(5): 1194-1200 (1998); Trei et al., Emerg. Infect. Dis., 16(5): 769-775 (2010)). Infections in the immunocompromised are similar in scope, but more severe, particularly in transplant recipients (Lion, T., Clin. Microbiol. Rev., 27(3): 441-462 (2014)). HIV infection is also a risk factor for AdV infection and recently serotypes were isolated from this population (De Jong et al., J. Clin. Microbiol., 37(12): 3940-3945 (1999)). Transmission of adenoviruses can occur via inhalation of aerosol droplets, fecal-oral transmission, and contaminated fomites (Lynch, J.P and Kajon, A.E., Seminars in Respiratory and Critical Care Medicine, 37(4): 586-602 (2016)). More than 50 serotypes of adenoviruses have been identified and are classified as six distinct groups (A-F) based on their biochemical, immunological and morphological criteria (Robinson et al., Scientific Reports, 3: 1812 (2013)). Approximately one-third of the described serotypes are associated with human disease. Globally, serotypes 1 - 7, 21, and 41 are most commonly associated with human disease. Different serotypes display different tissue tropisms that correlate with clinical manifestations of infection (Lynch and Kajon 2016, *supra*). The spectrum of clinical disease differs with age, immune status and population characteristics. Upper respiratory tract infections (URI) are among the most common, while pneumonia and diarrhea are the deadliest in infants. Epidemic keratoconjunctivitis and acute respiratory distress syndrome (ARDS) are the main concerns in young military recruits. Adenoviruses account for at least 5 - 10% of pediatric and 1 - 7% of adult respiratory tract infections (Lee et al., Journal of Medical Virology, 82(4): 624-631 (2010)). Pneumonia occurs in up to 20% of newborns and infants (Zhang et al., Infectious diseases, 48(1): 40-47 (2016); Jain et al., The New England Journal of Medicine, 372(9): 835-845 (2015)). Adenoviruses also account for > 50% of pneumonia cases among unvaccinated military recruits, not only in the US but also globally (Lynch and Kajon 2016, *supra).* In immunocompromised individuals, dissemination and/or severe respiratory failure develops in 10 - 30% of cases and fatality rates for severe adenoviral pneumonia may exceed 50% (Low et al., Respiratory Medicine, 107(11): 1810-1813 (2013); Ison, M.G., Clinical Infectious Diseases, 43(3): 331-339 (2006)). Very recently, an outbreak of AdV3 infection was reported in New Jersey at a nursing and rehabilitation center that resulted in 54 illnesses and 11 deaths. (See, https://nj.gov/health/cd/topics/adenovirus.shtml).

The options for controlling AdV infections are very limited. Live oral vaccines reduce the risk of respiratory illness and are in routine use by the US military, but currently are not available to civilians and only provide protection against types 4 and 7 (Radin et al., Clinical Infectious Diseases, 59(7): 962-968 (2014)). No antiviral drug has been approved for treating adenoviruses. Previous reports have shown specific antiviral activity for a number of nucleoside analogues in cell culture and animal models, including ribavirin, ganciclovir (GCV), cidofovir (CDV) and its prodrug brincidofovir (BCDV) (Hartline et al., The Journal of Infectious Diseases, 191(3): 396-399 (2005); Naesens et al., Antimicrobial Agents and Chemotherapy, 49(3): 1010-1016 (2005); Ying et al., Antimicrobial Agents and Chemotherapy, 58(12): 7171-7181 (2014); Toth et al., Antiviral Research, 153: 1-9 (2018); Toth et al., Proc. Nat. Acad. Sci. USA, 105(20): 7293-7297 (2008)). Ribavirin is teratogenic, causes hemolytic anemia and is active only against group C adenoviruses (Feld et al., Liver International, 37(1): 5-18 (2017); Morfin et al., Antiviral Therapy, 10(2): 225-229 (2005)). Ribavirin treatment of adenovirus infection in immunocompromised hosts yielded mixed results, which could be correlated to its serotype specificity (Ljungman, P., Eur. J. Clin. Microbiol. Infect. Dis., 23(8): 583-588 (2004); Lankester et al., Clinical Infectious Diseases, 38(11): 1521-1525 (2004)). The potency of GCV against AdV5 is very modest (IC50 = 66 ± 15.1 µM), and no controlled trials exist that indicate clinical efficacy for the use of GCV (Kinchington et al, The Journal of Antimicrobial Chemotherapy, 55(4): 424-429 (2005)). Long-term use of CDV has been associated with nephrotoxicity and CNS side effects (Bhadri et al., Transplant Infectious Disease, 11(4): 373-379 (2009); Vora et al., Journal of the Pediatric Infectious Diseases Society, 6(4): 399-402 (2017)). Brincidofovir (BCDV), a lipid-linked derivative of CDV, is orally bioavailable, highly potent *in vitro* and is currently undergoing clinical trials (Hartline et al. 2005, *supra*; Waye, M.M.Y. and Sing, C.W., Pharmaceuticals, 3(10): 3343-3354 (2010)). However, these trials have only shown modest benefit for AdV disease or viremia (Lopez et al., Current opinion in organ transplantation, 23(4): 395-399 (2018)). In a recent randomized placebo-controlled Phase 2 trial, the pre-emptive treatment of AdV infections with BCDV was shown to rapidly reduce viral loads (Grimley et al., Biology of Blood and Marrow Transplantation, 23(3): 512-521 (2017)). However, GI toxicity and acute kidney injury in solid organ transplant (SOT) and graft vs. host disease in hematopoietic cell transplant (HSCT) patients were reported (Detweiler et al., Journal of Pediatric Hematology/Oncology, 40(6): e364-e368 (2018); Faure et al., Medicine, 95(44): e5226 (2016)). Two Phase 3 AdV trials were completed several years ago (NCT011143181, NCT02087306) however, the company has not sought approval. Development of BCDV for HCMV has been discontinued because of the increased frequency of serious adverse events (primarily graft vs. host disease) and 24-week all-cause mortality observed during Phase 3 (Marty et al., Biology of Blood and Marrow Transplantation, 25(20: 369-381 (2018)). In addition, two Phase 3 clinical trials for CDV in kidney transplant patients were recently terminated (NCT02439957, NCT02439970). These safety issues call into question whether the drug can be administered safely in any population.
In the state of the art, a novel automated in vitro assay protocol for the high throughput screening of compounds to quantitate their antiviral activity and cytotoxicity in a single 384-well assay plate format, is disclosed. However, the authors note that these preliminary results should not be, without more, taken as definitive proof of a compound's actual inhibitory effectiveness (Hartline et al, Antiviral Research 159, 104-112 (2018).
In another study pharmacokinetics of MBX-400 in healthy volunteers are described for the purpose of developing an effective treatment for cytomegalovirus infection (Anonymous, Clinical Trials.gov., 2, (2017).

Currently there is no drug approved for the prevention or treatment of adenovirus infection and the drug furthest in development has safety issues that will likely preclude its advancement. Therefore, there exists an urgent and unmet medical need for a safe and effective anti-adenovirus drug for use in a method for treating and/or preventing adenovirus infections.

### Summary of the Invention

The present invention is directed to filociclovir (FCV) or a pharmaceutically acceptable salt thereof for use in a method of treating adenoviral infections . As described herein Filociclovir is suitable for use in a method of treating adenoviral infections in mammals, in particular humans.

In particular, filociclovir or a pharmaceutically acceptable salt thereof for use in the present method is directed to treating infection by specific strains of adenovirus. Therefore, in one embodiment, the present invention is directed to filociclovir or a pharmaceutically acceptable salt thereof for use in a method of treating infection in a mammal by adenovirus 5 (AdV5). In a preferred embodiment, the mammal is a human.

In another embodiment, the present invention is directed to filociclovir or a pharmaceutically acceptable salt thereof for use in a method of treating infection in a mammal by adenovirus 6 (AdV6). In a preferred embodiment, the mammal is a human.

In another embodiment, the present invention is directed to filociclovir or a pharmaceutically acceptable salt thereof for use in a method of treating infection in a mammal by adenovirus 8 (AdV8). In a preferred embodiment, the mammal is a human.

Also disclosed is filociclovir in the manufacture of a medicament of treating or preventing adenovirus infection in a mammal. Further disclosed is the use of filociclovir in the manufacture of a medicament to treat or prevent infection by AdV5, AdV6, and/or AdV8.

Also disclosed are pharmaceutical compositions comprising a therapeutically effective amount of filociclovir, or a pharmaceutically acceptable salt thereof, and a pharmaceutically acceptable carrier. The pharmaceutical compositions are suitable for use in the disclosed methods for treating or preventing adenoviral infections in a mammal, particularly humans. The pharmaceutical compositions may be formulated for both parenteral and/or nonparenteral administration to a subject or patient in need thereof.

Disclosed is futher that filociclovir may be administered to a subject in need thereof optionally in combination with one or more known antiviral agents. The additional antiviral agent or agents may be administered before, simultaneously with, or after administration of the filociclovir.

Also disclosed is that the filociclovir compound/composition of the present invention exhibits an inhibitory concentration of ≤10µM against infection by adenovirus and a cytotoxicity (CC₅₀) of ≥100-150µM.

### Definitions

A composition or method described herein as "comprising" (or "comprises") one or more named elements or steps is open-ended, meaning that the named elements or steps are essential, but other elements or steps may be added within the scope of the composition or method. To avoid prolixity, it is also understood that any composition or method described as "comprising" one or more named elements or steps also describes the corresponding, more limited, composition or method "consisting essentially of" (or "consists essentially of") the same named elements or steps, meaning that the composition or method includes the named essential elements and may also include additional elements or steps that do not materially affect the basic and novel characteristic(s) of the composition or method. It is also understood that any composition or method described herein as "comprising" or "consisting essentially of" one or more named elements or steps also describes the corresponding, more limited, and closed-ended composition or method "consisting of" (or "consists of") the named elements or steps to the exclusion of any other element or step. In any composition or method disclosed herein, known or disclosed equivalents of any named essential element or step may be substituted for that element or step, respectively.

Filociclovir (also referred to as cyclopropavir, 2-amino-9- {(Z)-[2,2-bis(hydroxymethyl)cyclopropylidene]methyl}-3,9-dihydro-6H-purin-6-one), and salts thereof, is a methylenecyclopropane nucleoside analogue represented by the following structure:

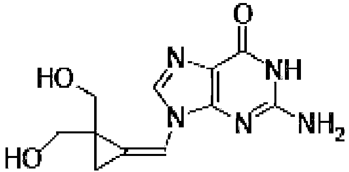

As used herein, the term "subject" can be a human, non-human primate, horse, pig, rabbit, dog, sheep, goat, cow, cat, guinea pig or rodent. A "patient" or "subject in need thereof" refers to a mammal afflicted with a disease or disorder. The term "patient" includes human and veterinary subjects.

Terms such as "parenteral", "parenterally", and the like, refer to routes or modes of administration of a compound or composition to an individual other than along the alimentary canal. Examples of parenteral routes of administration include, without limitation, subcutaneous (s.c.), intravenous (i.v.), intramuscular (i.m.), intra-arterial (i.a.), intraperitoneal (i.p.), transdermal (absorption through the skin or dermal layers), nasal ("intranasal"; absorption across nasal mucosa), or pulmonary (e.g., inhalation for absorption across the lung tissue), vaginal, direct injections or infusions into body cavities or organs other than those of the alimentary canal, as well as by implantation of any of a variety of devices into the body (e.g., of a composition, depot, or device that permits active or passive release of a compound or composition into the body).

The terms "non-parenteral", "non-parenterally", "enteral", "enterally", "oral", "orally", and the like, refer to administration of a compound or composition to an individual by a route or mode along the alimentary canal. Examples of enteral routes of administration include, without limitation, oral, as in swallowing solid (e.g., tablet) or liquid (e.g., syrup) dosage forms, sublingual (absorption through the mucosal membranes lining the floor of the mouth, e.g., under the tongue), buccal (absorption through the mucosal membranes lining the cheeks), nasojejunal or gastrostomy tubes (delivery into the stomach), intraduodenal administration, as well as rectal administration (e.g., suppositories for release of a drug composition into and absorption by the lower intestinal tract of the alimentary canal).

### Brief Description of the Drawings

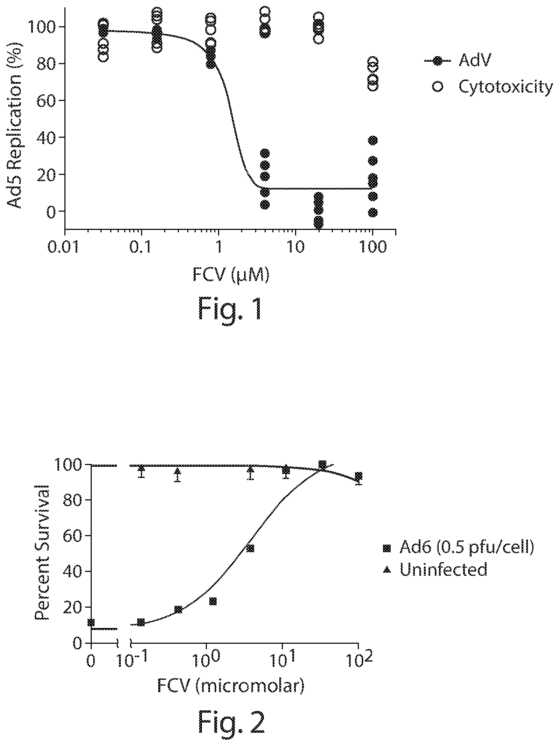

**Figure 1** is a dose response curve showing antiviral activity (closed symbols) and cytotoxicity (open symbols) of filociclovir against adenovirus 5 (AdV5).
**Figure. 2** is a dose response curve showing the antiviral activity and cytotoxicity of filociclovir against adenovirus 6 (AdV6).

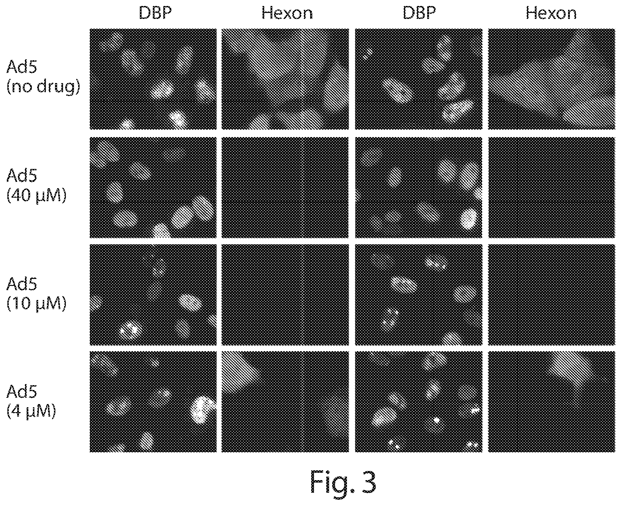

**Figure 3** is an immunofluorescence assay of A549 cells infected with adenovirus 5 and treated with filociclovir.

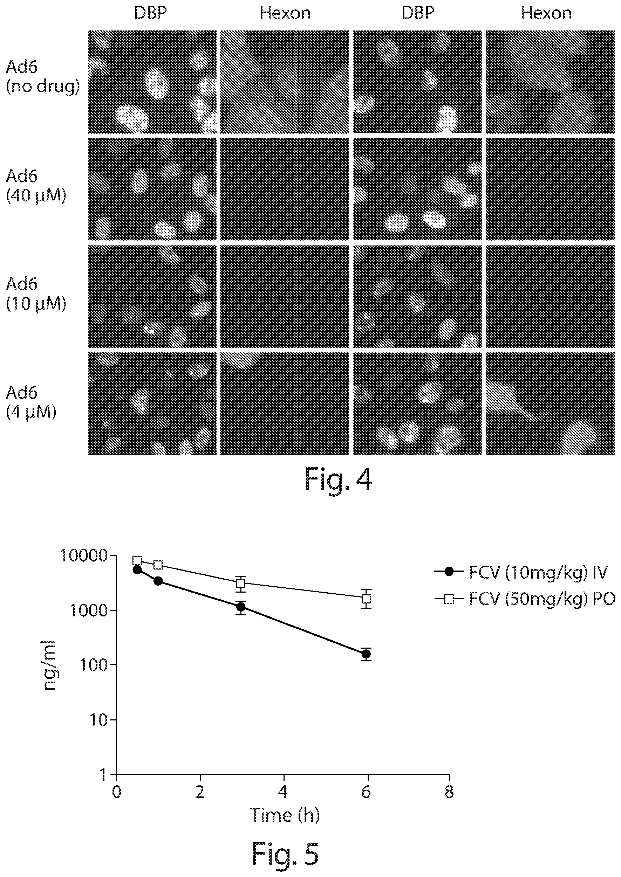

**Figure 4** is an immunofluorescence assay of A549 cells infected with adenovirus 6 and treated with filociclovir.
**Figure 5** shows the bioavailability of filociclovir administered orally (PO) or intravenously (IV) to hamsters.

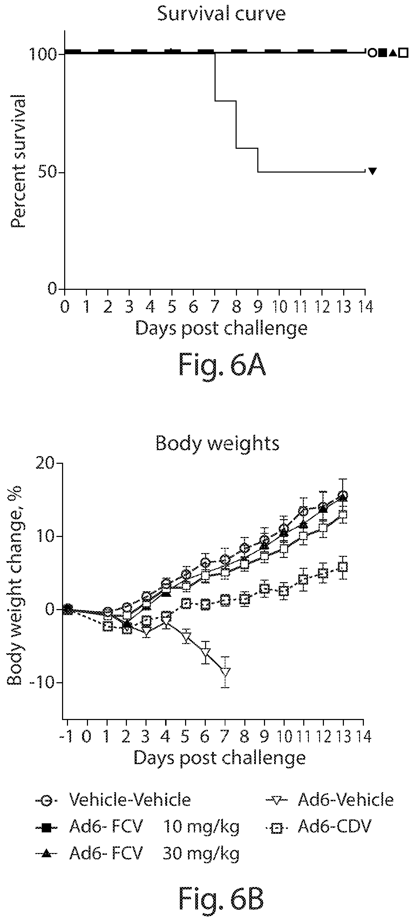

**Figures 6A** and **6B** show the percent survival (6A) and mean body weight changes (6B) of Syrian hamsters infected with AdV6 and treated with 10mg/kg, 30mg/kg, 60mg/kg, or 100mg/kg filociclovir. 6A: Survival. AdV6 + Vehicle vs. AdV6 + 10mg/kg or 30 mg/kg filociclovir (p=0.0124) (log rank). 6B: Mean body weight changes. Group means and standard error of the mean are shown. After moribund animals were sacrificed from a group, there were no means calculated for that group. AdV6 + Vehicle vs. AdV6 + 10mg/kg or 30 mg/kg filociclovir (p<0.0001( (two-way ANOVA); AdV6 + 10 mg/kg filociclovir at vs. Vehicle + Vehicle or AdV6 + 30 mg/kg filociclovir (p=0.0286).

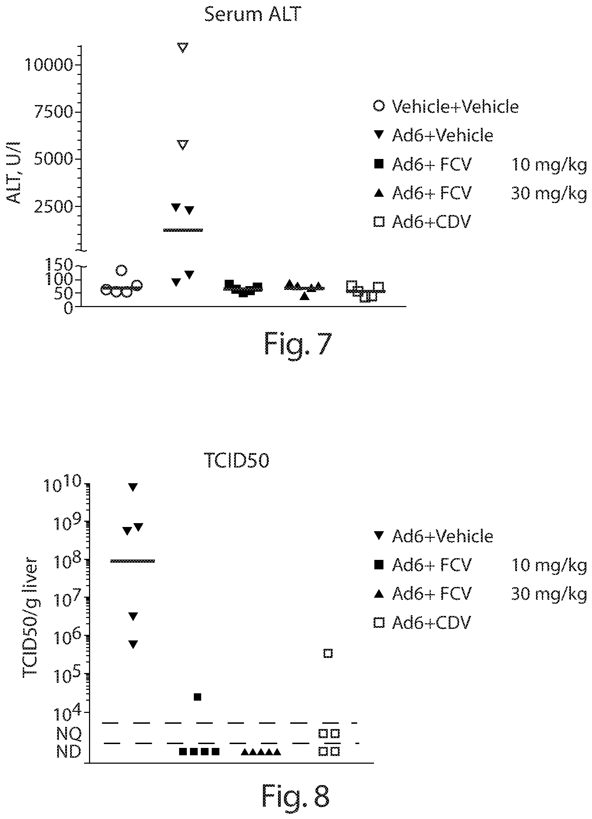

**Figure 7** shows the transaminase (ALT) levels of the Syrian hamsters at 5 days post challenge. Treatment with filociclovir mitigates liver pathology. The symbols indicate values from individual animals; the horizontal bar represents the geometric mean. The empty symbols in the AdV6 + Vehicle group denote samples that were collected from moribund animals.
**Figure 8** shows the level of AdV burden in the livers of the Syrian hamsters 5 days post challenge. Treatment with filociclovir inhibits AdV6 replication in the liver. The symbols indicate values from individual animals; the horizontal bar represents the geometric mean. NQ: not quantifiable; ND: not detectable.

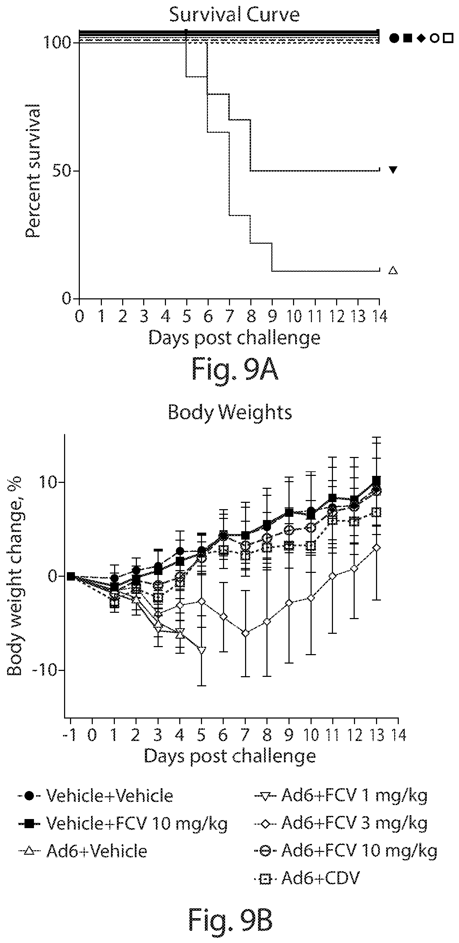

**Figures 9A** and **9B** show the percent survival (9A) and mean body weight changes (9B) of Syrian hamsters infected with AdV6 and treated with 1mg/kg, 3mg/kg, or 10mg/kg filociclovir. Filociclovir completely prevented mortality at the 3 and 10 mg/kg q.d. p.o. dose levels and had a moderate yet significant (p=0.0449) effect at the 1 mg/kg p.o. q.d. dose level (see, Figure 9A). Filociclovir completely prevented body weight loss at 10 mg/kg, was moderately efficacious at 3 mg/kg, and was not efficacious at 1 mg/kg. (See, Fig. 9B). No toxic effects were observed in the filociclovir treated hamsters. The symbols represent the group mean; the error bars signify the standard deviation. Body weight data for a given group are shown only up to time points at which no unscheduled deaths occurred.

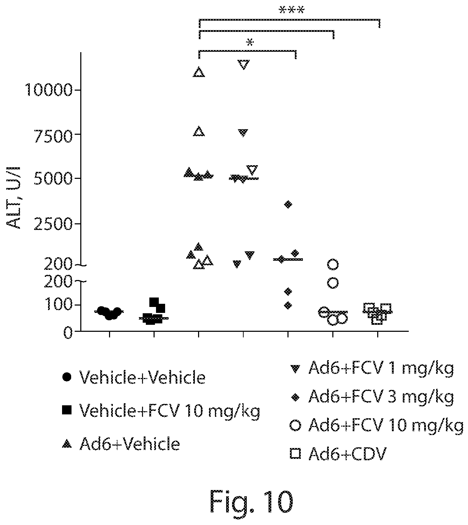

**Figure 10** shows the serum alanine aminotransferase (ALT) levels of the Syrian hamsters at 5 days post challenge. Filociclovir mitigated the pathology caused by intravenous injection of AdV6 at the 3 and 10 mg/kg q.d. p.o. dose levels, while it is ineffective at the 1 mg/kg p.o. q.d. dose level. The symbols represent the data collected from individual hamsters; the horizontal bar signifies the group mean. For the AdV6 + Vehicle and AdV6 + 1 mg/kg filociclovir groups, the empty symbols indicate that the samples were collected from hamsters sacrificed ahead of schedule. (*: p=0.0415; ***: p=0.0005) (one-tailed Mann-Whitney U-test)

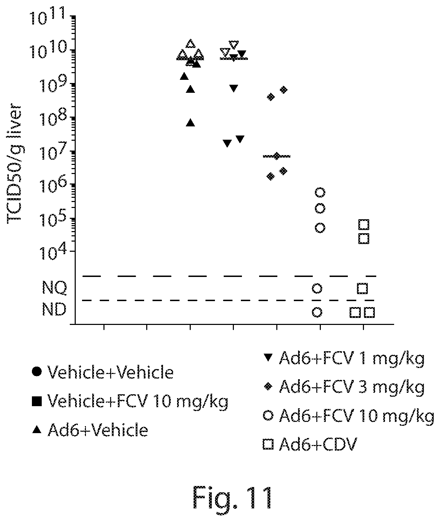

**Figure 11** shows the level of AdV burden in the livers of the Syrian hamsters 5 days post challenge. Treatment with 1, 3, or 10mg/kg filociclovir inhibits AdV6 replication in the liver. The symbols indicate values from individual hamsters; the horizontal bar represents the geometric mean. NQ: not quantifiable; ND: not detectable.

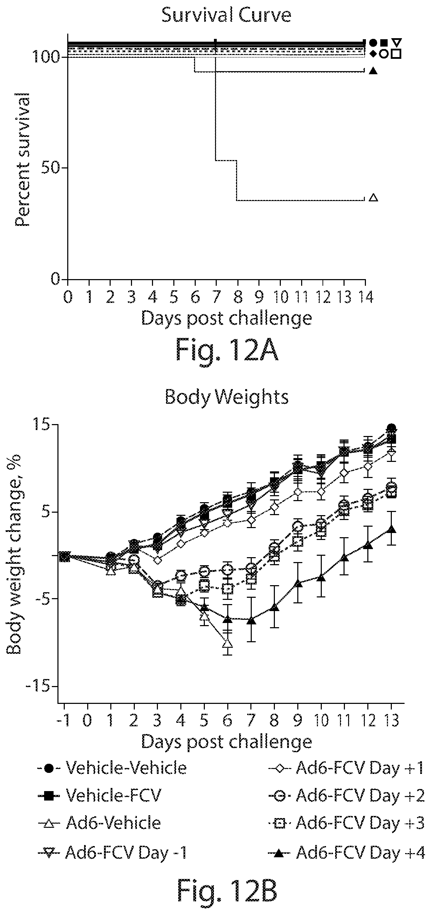

**Figures 12A and 12B** show the effects of delayed treatment with 10mg/kg filociclovir is efficacious against AdV6 infections in hamsters. 12A. Survival. AdV6 + Vehicle vs. AdV6 + filociclovir, day -1; AdV6 + filociclovir, day +1; AdV6 + filociclovir, day +2; or AdV6 + filociclovir, day +3 (p=0.0005); and AdV6 + Vehicle v. AdV6 + filociclovir, day +4 (p=0.0023) (log rank test); **12B.** Body weight changes. The symbols represent the group mean; the error bars signify the standard error of the mean.

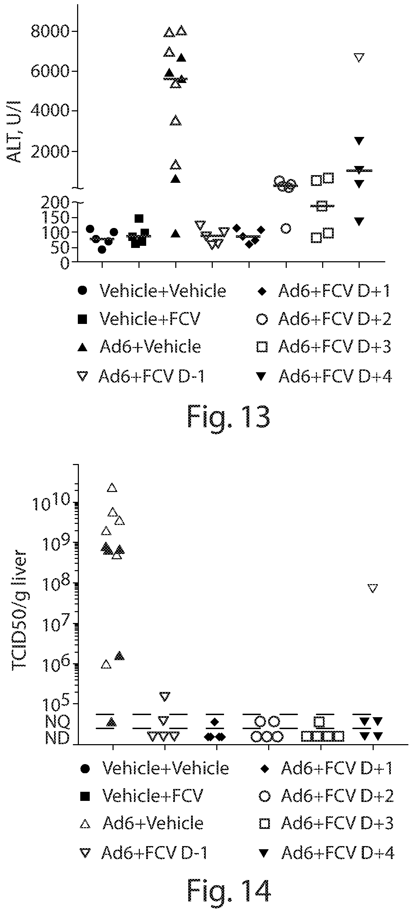

**Figure 13** shows the transaminase (ALT) levels of the Syrian hamsters at 1, 2, 3, and 4 days post challenge treatment with 10mg/kg filociclovir. Treatment with 10mg/kg filociclovir up to 4 days post challenge mitigates liver pathology. AdV6 + Vehicle vs. AdV6 + filociclovir, day -1 or AdV6 + filociclovir, day +1 (p=0.0018); AdV6 + Vehicle vs. AdV6 + filociclovir, day +2 (p=0.0087); AdV6 + Vehicle vs. AdV6 + filociclovir, day +3 (p=0.0032); and AdV6 + Vehicle vs. AdV6 + filociclovir, day +4 (p=0.1452) (two-tailed Mann-Whitney test) The symbols indicate values from individual animals; the horizontal bar represents the geometric mean. The empty symbols in the AdV6 + Vehicle group denote samples that were collected from moribund animals.
**Figure 14** shows the level of AdV burden in the livers of Syrian hamsters 1, 2, 3, and 4 days post challenge treated with 10mg/kg filociclovir. Treatment with 10mg/kg filociclovir 4 days post challenge inhibits AdV6 replication in the liver. The symbols indicate values from individual animals; the horizontal bar represents the geometric mean. NQ: not quantifiable; ND: not detectable.

### Detailed Description of the Invention

Human adenoviruses belong to the adenoviridae family, which is distinct from the herpesviridae family. For example, adenoviruses are double-stranded DNA viruses but, unlike herpesviruses, they are non-enveloped with an icosahedral nucleocapsid, and carry elongated fiber proteins on their outer surface. Adenoviruses are classified into seven species designated human adenovirus A to G. These viruses mainly infect the upper or lower respiratory tracts, conjunctiva, or gastrointestinal tract, causing a whole array of clinical manifestations that are distinct from those caused by herpesviruses. Most infections occur in children and immunocompromised individuals. Adenoviruses are not known to encode virus-encoded kinases, which implies a novel mechanism for filociclovir activation.

Other rare manifestations of AdV infections include hepatitis, as well as genitourinary and neurologic symptoms (Khanal et al. 2018, *supra*). The options for controlling AdV infections are very limited. Live oral vaccines reduce the risk of respiratory illness and are in routine use by the US military, but currently are not available to civilians and only provide protection against types 4 and 7 (Radin et al. 2014, *supra*). No antiviral drug has been approved for treating adenoviruses.

Herein we describe the discovery of the novel use of a filociclovir (FCV) or a pharmaceutically acceptable salt thereof in a method of treating adenovirus infection in a mammal. Our results demonstrate that filociclovir is unexpectedly effective for use in a method of treating adenovirus infection and is more effective at treating adenovirus infection than previously known treatments.

According to the invention filociclovir f is particularly effective for use in a method of treating adenovirus infection against a number of strains of adenovirus including, but not limited to adenovirus 3 (AdV3), adenovirus 4 (AdV4), adenovirus 5 (AdV5), adenovirus 6 (AdV6), adenovirus 7 (AdV7), adenovirus 8 (AdV8), adenovirus 19 (AdV19), and adenovirus 37 (AdV37).

Filociclovir or a pharmaceutically acceptable salt thereof is particularly suited for use in a method of treating adenovirus infections involving the upper respiratory tract and adenoviral infections of the eye, e.g., conjunctivitis, and in particular, keratoconjunctivitis involving the ocular surface of the eye.

The present invention is directed to filociclovir for use in a method of treating adenovirus infection in a mammalian subject. In a preferred embodiment, the mammal is a human.

The data described herein demonstrate that filociclovir is an effective pan-adenoviral therapeutic; thus, therapeutic administration of filociclovir is effective to treat or prevent infection by multiple strains of adenovirus. As described below, using several assays, it is demonstrated that filociclovir is a potent, safe, and effective inhibitor of a number of strains of adenovirus. For example, as set forth in Example 1, we used a cytopathic effects (CPE) reduction assay to demonstrate that filociclovir is a more potent inhibitor of adenovirus 5 than a number of other known viral inhibitors including cidofovir and ganciclovir (see, Table 1 and Fig. 1).

Next we used a neutral red survival assay to demonstrate that filociclovir is effective to reduce the cytotoxic effects of adenovirus 6. The results are shown in Fig. 2. Essentially, healthy cells are able to take up the neutral red dye via active transport into their lysosomes and virus infected cells are unable to take up the neutral red dye. The results shown in Fig. 2 demonstrate that filociclovir concentrations as low as 10⁻¹ µM were effective to improve viability of AdV6 infected cells, i.e., demonstrate active transport of the neutral red dye into the lysosomes of AdV6 infected cells.

As described in Example 3, an immunofluorescence stain assay was used to demonstrate that filociclovir is effective at inhibiting 'late' replication of AdV in infected human A549 cells. In this assay, cells were infected with either AdV5 or AdV6 or mock-infected. After 1 hour, filociclovir was added to a final concentration of 0, 4, 10, or 40 µM. The results are shown in Figs. 3 and 4. As seen in Figures 3 and 4, the results of filociclovir treatment was the same for AdV5 and AdV6 infection. Filociclovir was very effective at preventing late infection as demonstrated by the lack of presence of the hexon protein, a viral coat protein produced late in the adenovirus replication cycle. No hexon-positive cells were seen in the 40µM filociclovir treated wells and extremely few hexon-positive cells in the 10uM filociclovir treated wells. Some hexon-positive cells are seen in the 4µM filociclovir treated wells, but the 4µM wells show substantially less advanced infection (as indicated by the DBP staining patterns and the size and shape of nuclei) in comparison to the control (no drug) wells.

Using a Syrian hamster model of adenovirus infection as shown in Examples 4-7 , it is demonstrated that filociclovir exhibits favorable bioavailability when administered both orally and intravenously (see, Figure 5) and treatment of immunocompromised hamsters with doses of filociclovir ranging from 10mg/kg-100mg/kg following infection with AdV5 or AdV6, prevents morbidity and mortality (see, Fig. 6), mitigates liver damage (see, Fig. 7), and inhibits AdV virus replication (see, Fig. 8).

In Example 6, using the same Syrian hamster model described above, we also demonstrate that following AdV infection, even low doses of filociclovir (3mg/kg and 10mg/kg) are effective to prevent morbidity and mortality (Fig. 9), mitigate liver damage (Fig. 10), and inhibits AdV virus replication (Fig. 11).

In Example 7 it is shown that late administration of filociclovir (10mg/kg), e.g., up to 4 days post-challenge, is effective to prevent morbidity and mortality (see, Fig. 12), mitigate liver damage (see, Fig. 13), and inhibit AdV virus replication (see, Fig. 14) in Syrian hamsters infected with AdV6.

Therefore, in one embodiment, the present invention is directed to filociclovir for use in a method of treating infection of a mammal by adenovirus 5 (AdV5).

In another embodiment, the present invention is directed to filociclovir for use in a method of treating infection of a mammal by adenovirus 6 (AdV6).

In another embodiment, the present invention is directed to filociclovir for use in a method of treating infection of a mammal by adenovirus 7 (AdV7).

In yet another embodiment, the present invention is directed to filociclovir for use in a method of treating infection of a mammal by adenovirus 8 (AdV8).

Results also demonstrate that filociclovir is effective to treat or prevent infection of a mammal by adenovirus 3 (AdV3), adenovirus 4 (AdV4), adenovirus 19 (AdV19), and adenovirus 37 (AdV37). (Data not shown).

In a preferred embodiment, the mammal is a human.

Disclosed is also the use of filociclovir in a method for the manufacture of a medicament for treating or preventing adenovirus infection in mammals (e.g., humans) comprising combining filociclovir or a composition comprising filociclovir with a pharmaceutically acceptable carrier or diluent. Further disclosed is a method for manufacturing a medicament comprising combining at least one disclosed compound, e.g., filociclovir, according to the present invention or at least one disclosed product with a pharmaceutically acceptable carrier or diluent.

Individual dose requirements for administration to a subject will be determined by the caregiver, for example, a physician, primary care physician, or nurse.

As described herein, it has been demonstrated that filociclovir exhibits a dose-dependent inhibition of adenovirus infection with IC₅₀ values ≤5 µM and a minimal mammalian cytotoxicity (CC₅₀) of preferably ≥100-150µM.

Filociclovir can be administered as a pharmaceutically acceptable salt. Such pharmaceutically acceptable salts include the gluconate, lactate, acetate, tartarate, citrate, phosphate, maleate, borate, nitrate, sulfate, and hydrochloride salts. The salts of the compounds described herein can be prepared, for example, by reacting the base compound with the desired acid in solution. After the reaction is complete, the salts are crystallized from solution by the addition of an appropriate amount of solvent in which the salt is insoluble. In some embodiments, the hydrochloride salt is made by passing hydrogen chloride gas into an ethanolic solution of the free base.

Disclosed are also compounds, which are formulated into a pharmaceutically acceptable carrier or excipient for administration to a subject in need thereof. The compounds may be formulated into a pharmaceutical formulation and further comprise an additional antiviral compound. The pharmaceutical formulation may be formulated to be administered orally, parenterally, or topically.

It is preferable to develop an orally active therapeutic, since that is the most convenient and rapid method to administer a drug to a large exposed population in case of pandemic. Currently, live oral adenovirus vaccines for protection against serotypes 4 and 7 are in use by the military but are not available to the civilian population. However, it is also expected that filociclovir as described herein will be suitable for intravenous (i.v.) administration, because it is envisioned that in case of a natural outbreak the infected patients may require i.v. administration. Therefore, the method described herein will provide an effective, safe, and easy therapeutic option for any newly emerged pandemic strain(s) of adenovirus.

The methods disclosed are useful for treating these conditions in that they inhibit the onset, growth, or spread of the condition, cause regression of the condition, cure the condition, or otherwise improve the general well-being of a subject afflicted with, or at risk of, contracting the condition. Thus, in accordance with the presently disclosed subject matter, the terms "treat", "treating", and grammatical variations thereof, as well as the phrase "method of treating", are meant to encompass any desired therapeutic intervention, including but not limited to a method for treating an existing adenovirus infection in a subject, and a method for the prophylaxis (i.e., preventing) of an adenovirus infection, such as in a subject that has been exposed to a virus as disclosed herein or that has an expectation of being exposed to a virus as disclosed herein.

Pharmaceutical compositions disclosed herein comprise the adenovirus inhibitor, filociclovir, as described herein, or a pharmaceutically acceptable salt thereof, as the 'active ingredient' and a pharmaceutically acceptable carrier (or 'vehicle'), which may be a liquid, solid, amorphous, or semi-solid compound.

Further disclosed is a method of treating or preventing an adenovirus infection in a subject in need of treatment thereof wherein the method comprises administering to the subject an effective amount of a composition comprising filociclovir. Filociclovir may be administered alone or optionally in combination with one or more additional antiviral agents.

Filociclovir may be formulated into a pharmaceutically-acceptable carrier and applied/administered to a subject in need thereof by an injection, including, without limitation, intradermal, transdermal, intramuscular, intraperitoneal and intravenous. As an example the administration is oral and the compound may be presented, for example, in the form of a tablet or encased in a gelatin capsule or a microcapsule, which simplifies oral application. The production of these forms of administration is within the general knowledge of a technical expert. Multiple routes of administration are envisioned for the method disclosed herein and highly cost-effective production strategies can be easily achieved.

Dsiclosed are also pharmaceutical compositions comprising filociclovir, or a pharmaceutically acceptable salt, solvate, hydrate, or polymorph thereof, and a pharmaceutically acceptable carrier.

The pharmaceutical composition is a solid dosage form selected from a capsule, a tablet, a pill, a powder, a granule, an effervescing granule, a gel, a paste, a troche, and a pastille. The pharmaceutical composition is a liquid dosage form selected from an emulsion, a solution, a suspension, a syrup, and an elixir.

As used herein, the term "pharmaceutically acceptable salts" refers to salts prepared from pharmaceutically acceptable non-toxic bases or acids. When the compound disclosed herein is acidic, its corresponding salt can be conveniently prepared from pharmaceutically acceptable non-toxic bases, including inorganic bases and organic bases. Salts derived from such inorganic bases include aluminum, ammonium, calcium, copper (-ic and -ous), ferric, ferrous, lithium, magnesium, manganese (-ic and -ous), potassium, sodium, zinc and the like salts. Salts derived from pharmaceutically acceptable organic non-toxic bases include salts of primary, secondary, and tertiary amines, as well as cyclic amines and substituted amines such as naturally occurring and synthesized substituted amines. Other pharmaceutically acceptable organic non-toxic bases from which salts can be formed include ion exchange resins such as, for example, arginine, betaine, caffeine, choline, N,N'-dibenzylethylenediamine, diethylamine, 2-diethylaminoethanol, 2-dimethylaminoethanol, ethanolamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and the like.

As used herein, the term "pharmaceutically acceptable non-toxic acids", includes inorganic acids, organic acids, and salts prepared therefrom, for example, acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid and the like. Preferred are citric, hydrobromic, hydrochloric, maleic, phosphoric, sulfuric, and tartaric acids.

In preparing the compositions for oral dosage form, any convenient pharmaceutical media can be employed. For example, water, glycols, oils, alcohols, flavoring agents, preservatives, coloring agents and the like can be used to form oral liquid preparations such as suspensions, elixirs and solutions; while carriers such as starches, sugars, microcrystalline cellulose, diluents, granulating agents, lubricants, binders, disintegrating agents, and the like can be used to form oral solid preparations such as powders, capsules and tablets. Because of their ease of administration, tablets and capsules are the preferred oral dosage units whereby solid pharmaceutical carriers are employed. Optionally, tablets can be coated by standard aqueous or nonaqueous techniques

A tablet containing the composition herein disclosed can be prepared by compression or molding, optionally with one or more accessory ingredients or adjuvants. Compressed tablets can be prepared by compressing, in a suitable machine, the active ingredient in a free-flowing form such as powder or granules, optionally mixed with a binder, lubricant, inert diluent, surface active or dispersing agent. Molded tablets can be made by molding in a suitable machine, a mixture of the powdered compound moistened with an inert liquid diluent.

Pharmaceutical compositions disclosed herein are suitable for parenteral administration can be prepared as solutions or suspensions of the active compounds in water, from, for example, lyophilized samples of the active compounds. A suitable surfactant can be included such as, for example, hydroxypropylcellulose. Dispersions can also be prepared in glycerol, liquid polyethylene glycols, and mixtures thereof in oils. Further, a preservative can be included to prevent the detrimental growth of microorganisms.

Pharmaceutical compositions disclosed herein are suitable for injectable use include sterile aqueous solutions, emulsions, or dispersions. Furthermore, the compositions can be in the form of sterile powders for the extemporaneous preparation of such sterile injectable solutions or dispersions. In all cases, the final injectable form must be sterile and must be effectively fluid for easy syringability. The pharmaceutical compositions must be stable under the conditions of manufacture and storage; thus, preferably should be preserved against the contaminating action of microorganisms such as bacteria and fungi. The carrier can be a solvent or dispersion medium containing, for example, water, ethanol, polyol (*e.g.,* glycerol, propylene glycol and liquid polyethylene glycol), vegetable oils, and suitable mixtures thereof.

Pharmaceutical compositions herein disclosed can be in a form suitable for topical use such as, for example, an aerosol, cream, ointment, lotion, dusting powder, mouth washes, gargles, and the like. Further, the compositions can be in a form suitable for use in transdermal devices. These formulations can be prepared, utilizing a compound of the invention, or pharmaceutically acceptable salts thereof, via conventional processing methods. As an example, a cream or ointment is prepared by mixing hydrophilic material and water, together with about 5 wt% to about 10 wt% of the compound, to produce a cream or ointment having a desired consistency.

Pharmaceutical compositions disclosed herein can be in a form suitable for rectal administration wherein the carrier is a solid. It is preferable that the mixture forms unit dose suppositories. Suitable carriers include cocoa butter and other materials commonly used in the art. The suppositories can be conveniently formed by first admixing the composition with the softened or melted carrier(s) followed by chilling and shaping in molds.

In addition to the aforementioned carrier ingredients, the pharmaceutical formulations described above can include, as appropriate, one or more additional carrier ingredients such as diluents, buffers, flavoring agents, binders, surface-active agents, thickeners, lubricants, preservatives (including anti-oxidants) and the like. Furthermore, other adjuvants can be included to render the formulation isotonic with the blood of the intended recipient. Compositions containing a compound of the invention, and/or pharmaceutically acceptable salts thereof, can also be prepared in powder or liquid concentrate form.

Further disclosed is a kit comprising at least filociclovir according to the present invention, or a pharmaceutically acceptable salt, solvate, or polymorph thereof; and one or more of:
a) optionally at least one additional agent known to have antiviral activity;
b) instructions for treating an adenovirus related disease;
c) instructions for administering the compound in connection with treating an adenovirus infection; and/or
d) instructions for administering the compound with at least one agent known to treat an adenovirus related disease.

The kits can also comprise compounds and/or products co-packaged, co-formulated, and/or co-delivered with other components. For example, a drug manufacturer, a drug reseller, a physician, a compounding shop, or a pharmacist can provide a kit comprising a disclosed compound of the present invention and/or product and another component for delivery to a patient.

In a further aspect, the kit further comprises a plurality of dosage forms, the plurality comprising one or more doses; wherein each dose comprises an amount of the compound and the agent known to have antiviral activity. In another aspect, the kit further comprises a plurality of dosage forms, the plurality comprising one or more doses; wherein each dose comprises an effective amount of the compound and the agent known to have antiviral activity.

In a further aspect, an effective amount is a therapeutically effective amount. In a still further aspect, an effective amount is a prophylactically effective amount.

### Examples

The following Examples have been included to illustrate modes of the presently disclosed subject matter. In light of the present disclosure and the general level of skill in the art, those of skill will appreciate that the following Examples are intended to be exemplary only and that numerous changes, modifications, and alterations can be employed without departing from the invention as presently disclosed.

### Example 1. Cytopathic Effects (CPE) reduction assay to measure inhibition of Adenovirus 5 (AdV5) by filociclovir

### Preparation and culture of human foreskin fibroblast (HFF) cells

Human foreskin tissue was obtained from the University of Alabama at Birmingham tissue procurement facility with approval from the Institutional Review Board. The tissue was stored at 4°C in cell culture medium consisting of minimum essential media (MEM) with Earle's salts supplemented with 10% fetal bovine serum (FBS; HyClone, Inc., Logan, UT) and standard concentration of L-glutamine, amphotericin B (Fungizone), and vancomycin. The tissue was then placed in a phosphate-buffered saline solution, minced, and rinsed to remove the red blood cells. Tissue fragments were then resuspended in a trypsin-EDTA solution and incubated at 37°C to disperse the cells, which were then collected by centrifugation. Cell pellets were then resuspended in 4 ml culture medium, placed in a 25-cm² tissue culture flask, and incubated at 37°C for 24h. The culture medium was then replaced with fresh medium and condition of the cells was monitored daily until a confluent cell monolayer was formed. The HFF cells were then expanded through serial passages in standard growth medium of MEM with Earle's salts supplemented with 10% FBS, L-glutamine, penicillin, and gentamicin. Each lot of cells was confirmed to be free of mycoplasma infection and routinely passaged or used for assays at or before passage 10.

### Antiviral Assay

Antiviral and cytotoxicity data were obtained in a series of three to five separate experiments for each virus to provide an accurate estimate of antiviral activity and statistical data. Each assay included positive and negative control compounds as well as infected and uninfected controls to ensure the integrity of the experiment. The concurrent assessment of cytotoxicity was performed in each assay plate using the same number of cells and equivalent levels of compound exposure so that accurate selective index (SI) values could be obtained. All liquid handling steps were performed on a BioMek 4000 and significantly increased the efficiency of the assays and reduced hands-on time of analysts.

The CPE reduction assays were performed in monolayers of human foreskin fibroblast (HFF) cells (5000/well) in 384-well plates in assay media consisting of MEM with Earle's salts, 2% FBS and standard concentrations of L-glutamine, penicillin and gentamycin. 5000 cells were seeded in 384-well microtiter plates and incubated at 37°C in a humidified 5% CO₂ incubator for 24 hours to allow for formation of confluent monolayers. Dilutions of antiviral test compounds, including filociclovir, were prepared directly in the plates in a series of 5-fold dilutions in duplicate wells to yield final concentrations that ranged from 0.1 to 300 µM or from 0.003 to 10 µM which allows for a large dynamic range of compound concentrations to facilitate the detection of antiviral activity of unknown compounds with either weak or potent antiviral activity.

Cell monolayers were infected at a multiplicity of infection (MOI) of approximately 0.005 PFU/cell with virus strains including AdV5. Infected cells were incubated at 37°C until 100% CPE was observed in the virus control wells. Cytopathology was determined by the addition of CellTiter-Glo^{®} reagent (Promega, Madison, WI). Concentrations of antiviral test compounds sufficient to reduce CPE by 50% (EC₅₀) were interpolated from the experimental data using standard methods in Microsoft^{®} Excel. Cytotoxicity was also determined with CellTiter-Glo^{®} luminescent cell viability assay (Promega, Madison, WI) and concentrations of the test compounds that decreased cell viability by 50% (CC₅₀) were calculated from the data and selective index (SI) values were calculated as the CC₅₀/EC₅₀ as a measure of antiviral activity. The results are shown in Table 1 below and Figure 1.

**Table 1 -- Cytotoxicity of various antiviral compounds against Adenovirus 5**

| Drug | AdV 384 Well CPE^{a} | | SI |
|---|---|---|---|
| | EC₅₀ | CC₅₀ | |
| CDV | 4.0 ± 2.0 | 97 ± 4 | 24.3 |
| PFA | >1000 ± 0 | >1000 ± 0 | 1.0 |
| GCV | 66 ± 15.1 | >100 ± 0 | 1.5 |
| ACV | >100 ± 0 | >100 ± 0 | 1.0 |
| PCV | >100 ± 0 | >100 ± 0 | 1.0 |
| FIAU | >100 ± 0 | >100 ± 0 | 1.0 |
| IDU | >100 ± 0 | >100 ± 0 | 1.0 |
| BDCRB | 70 ± 39 | 91 ± 13 | 1.3 |
| CMX001 | <0.03 ± 0 | 0.83 ± 0.7 | 27.7 |
| AZT | >100 ± 0 | >100 ± 0 | 1.0 |
| FCV | 2.2 ± 0.4 | 95 ± 10.6 | 43.2 |
| 4-thio-IDU | 11 ± 9.4 | 26 ± 24 | 2.4 |
| N-MCT | 57 ± 47 | 72 ± 33 | 1.3 |
| L-BHDU | >100 ± 0 | >100 ± 0 | 1.0 |
| PMEA | 48 ± 8 | >100 ± 0 | 2.1 |

| | | | |
|---|---|---|---|
| ^{a} Values shown represent the average EC₅₀ and CC₅₀ values (µM) and SD from five independent experiments. | | | |

### Abbreviations:

CDX - cidofovir; PFA - foscarnet; GCV - ganciclovir; ACV - acyclovir;
PCV - penciclovir; FIAU - fluoroiodoarabinosyl adenine; IDU - idoxuridine;
BDCRB - bromodichlororibobenzimidazole; CMX001 - brincidofovir; AZT - azidothymidine; FCV- filociclovir; 4-thio-IDU-4-thioidoxuridine; N-MCT-N-methanocarbathymidine; L-BHDU - L-Bromovinyl-Hydroxymethyl-Dioxolan Uracil;
PMEA - adefovir

The results shown in Table 1 demonstrate that filociclovir (FCV) exhibits significantly improved inhibitory activity against adenovirus 5 (SI = 43) over all the other test compounds and was at least twice as potent as the next most effective antiviral agent, cidofovir (CDV) (SI = 24) and significantly better than ganciclovir (GCV) (SI ≥ 1.5).

The percent reduction in AdV5 replication as a function of filociclovir concentration ranging from 0.01 to 100µM is shown in Figure 1. The results in Figure 1 demonstrate that concentrations of filociclovir as low as 0.2µM are effective to reduce AdV replication.

Similar experiments were carried out with filociclovir against adenovirus 6, adenovirus 7, and adenovirus 8. The results are shown below in Table 2. Filociclovir exhibited potent inhibition of adenovirus 8 (SI ≥ 61).

**Table 2 -- Cytotoxicity of Filociclovir**

| **Virus** | EC₅₀ | CC₅₀ | CC50/EC50 |
|---|---|---|---|
| **AdV6** | 3.67 | >100 | 27 |
| **AdV8** | 2.45 | >150 | 61 |
| **AdV7** | 1.04 | >150 | >144 |

### Example 2. Neutral Red Survival Assay to measure inhibition of Adenovirus 6 (AdV6) by filociclovir

The neutral red cell cytotoxicity assay was used to detect cell viability or drug cytotoxicity. The principle of this assay is based on the detection of viable cells via the uptake of the dye neutral red. Neutral red is a eurhodin dye that stains lysosomes in viable cells. Viable cells can take up neutral red via active transport and incorporate the dye into their lysosomes, but non-viable cells are unable not take up this chromophore. Consequently, after washing, viable cells can release the incorporated dye under acidified-extracted conditions. The amount of released dye can be quantified and used to determine the total number of viable cells or drug cytotoxicity. As such, cytotoxicity is expressed as a concentration-dependent reduction of the uptake of neutral red after exposure to the compound under investigation.

The neutral red survival assays were carried out in a 96-well format. Briefly, A549 adenocarcinomic human alveolar basal epithelial cells were plated at a concentration of 6×10³ cells per well one day prior to infection. AdV6 infections were carried out at 5×10³ PFU per well (an estimated 0.5 PFU/cell at the time of infection). Filociclovir was serially diluted 1:3 on separate 96-well plates with a no drug final row as control. The AdV6 infections were performed as 9 replicate wells for each drug concentration and there were 3 uninfected drug control wells for each drug concentration. Filociclovir dilutions were added to the cell plates immediately before infection with AdV6. At 6 days post-infection (when viral cytopathic effect had reached 70-90% for the virus-infected, no drug wells), neutral red was added for 1 hour, then plates were washed 3 times with PBS to remove unattached cells, and the neutral red was extracted from the remaining cells using 50% ethanol/1% glacial acetic acid. Filociclovir plates were read and the results were graphed using GraphPad Prism graphing and statistical software (GraphPad Software, San Diego, CA).

The results are shown in Figure 2 . As seen in Figure 2, filociclovir concentrations as low as 10⁻¹µM were effective to improve viability of AdV6 infected cells.

### Example 3. Immunofluorescence assay of filociclovir-treated AdV5 or AdV6 infections of human A549 Cells

Human A549 cells on glass coverslips in 6-well plates were infected at an MOI of 5 PFU/cell with either AdV5 or AdV6 or were mock-infected. After 1 hour, filociclovir was added to a final concentration of 0, 4, 10, or 40µM. At 27 hours post-infection, the A549 cells were fixed in paraformaldehyde (3.7% in PBS) and permeabilized with methanol. Cells were stained for the Adenovirus DNA-binding protein and adenovirus hexon. DBP staining will be antinuclear and uniform during early AdV infection (prior to AdV DNA replication). DBP associates with replication centers as the infection progresses. The replication centers are initially small "dots" (each dot results from one incoming AdV genome). The replication centers will expand and "multiply" as DNA replication takes place. The nuclei become enlarged and misshapen as infection progresses. AdV hexon, the most abundant component of the AdV viral capsid, is not expressed until after DNA replication is taking place and is therefore considered to be a "late" AdV protein. The results are shown in Figures 3 and 4.

As seen in Figures 3 and 4, the results of filociclovir treatment was the same for AdV5 and AdV6 infection. Filociclovir was very effective in preventing late infection (as would be indicated by hexon staining). No hexon-positive cells were seen for the 40 µM filociclovir wells and extremely few hexon-positive cells for the 10µM filociclovir wells. Some hexon-positive cells are seen with the 4µM filociclovir wells, but the 4µM wells show substantially less advanced infection (as indicated by the DBP staining patterns and the size and shape of nuclei) in comparison to the control (no drug) wells.

The results show that filociclovir is a potent inhibitor of human adenovirus 5 (AdV5), AdV6, and AdV8. This level of activity is similar to that of cidofovir (CDV) and far superior to that of ganciclovir (GCV), which has an IC₅₀ value of 66µM against AdV5. (See, Table 1) The range of AdV serotypes tested so far suggest that FCV could potentially be developed as a pan-adenoviral inhibitor.

### Example 4. Pharmacokinetics of Filociclovir

In order to test the pharmacokinetics of filociclovir, we use a Syrian male hamster model of adenovirus infection. Syrian hamsters were given either an oral (p.o. 50mg/kg) or intravenous (i.v. 10mg/kg) dose of filociclovir. The results are shown in Figure 5 and demonstrate that filociclovir was still detectable 6 hours after PO or IV administration.

### Syrian hamster AdV model

The Syrian male hamster model is particularly advantageous as it mimics the pathology seen with human patients, and it can be used to test the efficacy of antiviral compounds (reviewed in Wold, W.S.M. and Toth, K., Advances in Cancer Research, 115: 69-92 (2012)). Syrian hamsters are one of the two rodent species (the other is the cotton rat) that are permissive to AdV species C infections (types 1, 2, 5, and 6).

Using this model, it is possible to carry out controlled *in vivo* experiments to test the efficacy of anti-adenoviral compounds (Ying et al., Antimicrobial Agents and Chemotherapy, 58(12): 7171-7181 (2014); Toth et al., Proc. Natl. Acad. Sci. USA, 105(20): 7293-7297 (2008); Tollefson et al. 2014; Toth et al., Viruses, 7(3): 1409-1428 (2015)).

For these experiments, young hamsters were immunosuppressed with cyclophosphamide (CP), an agent often used as part of the conditioning regimen for human transplant recipients. After the desired degree of immunosuppression was achieved, the hamsters were infected intravenously (iv) with AdV5, leading to replication of AdV5 in most organs, most prominently in the liver (Toth et al. 2008 *supra*).

### Drug formulation

For p.o. administration, filociclovir was suspended in 0.4% carboxymethyl cellulose (Sigma C5678) at 5mg/ml and sonicated to visual homogeneity. For i.v. administration, filociclovir was dissolved in DMSO (Sigma D2650) and then diluted with water to a final concentration of 5mg/ml for filociclovir and 75% DMSO. The dosing solutions were prepared one day before use and stored at 4°C.

### Experimental design

Male Syrian hamsters were purchased from Envigo (Hackensack, NJ) at ~100g body weight. All hamsters were immunosuppressed using cyclophosphamide (CP) administered intraperitoneally at a dose of 140mg/kg, and then twice weekly at a dose of 100mg/kg. The animals received three injections of CP before the administration of filociclovir. Two groups of animals (12 hamsters/group) were used for the two routes of administrations. In addition, three untreated animals (the same three hamsters for both routes) served as mock controls. The p.o. and i.v. experiments were performed on two consecutive days. As the hamsters weighed approximately 100g, (+/-10%), they were administered 1ml and 0.2ml for the p.o. and i.v. routes, respectively, to achieve the desired dose. For both routes, 3 hamsters were sacrificed at 0.5, 1, 3, and 6 hr post dosing.

Plasma and liver samples were collected from all animals and stored at -80°C until analyzed by LC MS as follows:
- All samples and standards were processed on ice;
- Standard curve is constructed with authentic compound (fresh powder DMSO reconstitution spiked into "blank" Hamster plasma) and all samples spiked with 500 ng/mL carbamezapine (Internal Standard);
- Early time point samples are diluted in "blank" hamster plasma (try to attain concentrations on the standard curve);
- Sample extracted with 2X sample volume with 0.1% ammonium formate in methanol (i.e. 30µL sample + 60µL 0.1% ammonium formate in methanol);
- Extracted samples centrifuged on maximum setting in microfuge;
- Supernatants are analyzed by LC/MS/MS Dynamic Multiple Reaction Monitoring (DMRM) utilizing custom method.

### Example 5. Determining the Therapeutic Efficacy of High Dose Filociclovir Against Intravenously Administered Human AdV6 in Immunosuppressed Male Syrian Hamsters

As set forth above, it has been established that filociclovir is an effective inhibitor against several strains of adenovirus replication *in vitro* and demonstrates good oral bioavailability in hamsters.

Next, we assessed whether filociclovir exhibits anti-adenoviral efficacy in immunosuppressed Syrian hamsters infected intravenously (i.v.) with AdV6. Four dose levels were tested: 10, 30, 60, and 100mg/kg p.o. q.d. The highest dose was determined based on rat toxicology data.

### Experimental design

All hamsters were immunosuppressed using cyclophosphamide (CP). CP was administered intraperitoneally at a dose of 140mg/kg, and then twice weekly at a dose of 100 mg/kg.

Filociclovir, in powdered form, was suspended in 0.4% carboxymethyl cellulose (Sigma C5678) at 1, 3, 6, and 10mg/ml and sonicated to visual homogeneity and aliquots stored at 4°C. Aliquots were allowed to equilibrate to room temperature before dosing. Hamsters weighing approximately 100g were dosed with a 1ml volume of the appropriate suspension for the 10, 30, 60, and 100mg/kg dose levels. References to "vehicle" refers to 0.4% carboxymethyl cellulose

The hamsters were divided into 8 groups, 15 hamsters/group, (with the exception of Group 2, which had only 5 hamsters; see Table 3), immunosuppressed, and then injected i.v. with vehicle (Groups 1-2) or 2x10¹⁰ PFU/kg of AdV6 (Groups 5-9). Groups 1 and 3 received drug vehicle (p.o. q.d.), Group 4 received 10mg/kg filociclovir (p.o. q.d.), Group 5 received 30mg/kg filociclovir (p.o. q.d.), Group 6 received 60mg/kg filociclovir (p.o. q.d.), Groups 2 and 7 received 100mg/kg filociclovir (p.o. q.d.), and Group 8 received cidofovir (20mg/kg, 3 times weekly). For all groups, drug administration started 1 day before challenge, and continued according to the schedule above for the duration of the study.

The body weights and any signs of morbidity of the animals were recorded daily. At 5 days post challenge, 5 hamsters (designated at the start of the experiment) from each group (with the exception of Group 2) were sacrificed, and gross pathological observation was performed. Serum and liver samples were collected and the virus burden in liver was determined by a TCID50 assay, and the serum was analyzed for transaminase levels. The remaining 10 hamsters were sacrificed at 14 days post challenge. Hamsters that became visually moribund before Day 14 were sacrificed as needed. In addition to the hamsters observed to be moribund, all hamsters that lost more than 20% of their original body weight were also sacrificed. For the hamsters sacrificed at the conclusion of the experiment and those sacrificed as moribund, serum and liver tissues were collected and banked for possible determination of serum transaminase levels and virus burden in the liver. Liver tissue was preserved in formalin for possible histopathological examination.

**Table 3 --Therapeutic window study for Filociclovir - AdV6-infected male animals**

| **Group #** | **Groups** | **n** | **Work Performed** | | |
|---|---|---|---|---|---|
| | | | **Daily** | **At Necropsy** | |
| | | | | **Day 5** | **Day 14** |
| **1** | **Vehicle** | 5+10 | | Serum ALT, bank liver for histopathology | Bank serum for ALT, liver for histopathology |
| **2¹** | **Vehicle + filociclovir 100mg/kg p.o. q.d.** | 5 | | | Bank serum for ALT, liver for histopathology |
| **3** | **AdV6 + Vehicle** | 5+10 | | Serum ALT, liver TCID50, bank liver for histopathology | Bank serum for ALT, liver for TCID50, liver for histopathology |
| **4** | **AdV6 + filociclovir 10mg/kg p.o. q.d.** | 5+10 | | | |
| **5** | **AdV6 + filociclovir 30mg/kg p.o. q.d.** | 5+10 | Dose drug/drug vehicle daily, Observation, Body Weight | | |
| **6¹** | **AdV6 + filociclovir 60mg/kg p.o. q.d.** | 5+10 | | | |
| **7¹** | **AdV6 + filociclovir 100mg/kg p.o. q.d.** | 5+10 | | | |
| **8** | **AdV6 + CDV 20mg/kg i.p. 3x weekly** | 5+10 | | | |

| | | | | | |
|---|---|---|---|---|---|
| ¹ Removed from study | | | | | |

### Results

### In-life observations

There were 5 treatment-related deaths in the experiment, all in the AdV6 + Vehicle group (Fig. 6A). Filociclovir at 10 or 30mg/kg prevented mortality (Fig. 6A). Initially, all animals in the AdV6-infected groups lost body weight; this was reversed by treatment with 10mg/kg or 30 mg/kg filociclovir (Fig. 6B). The weight gain of animals in the AdV6 + 10mg/kg filociclovir was slightly lower than that of uninfected animals and animals in the AdV6 + 30mg/kg filociclovir group. Higher doses of filociclovir, especially the 100mg/kg dose, are toxic to AdV6-infected animals (not shown); these groups were removed from the study.

### Necropsy

At the day 5 (D5) sacrifice, extensive kidney pathology was observed for animals in the AdV6 + 60mg/kg and 100mg/kg filociclovir groups.

### Serum transaminase levels

At 5 days post challenge, serum was collected from 5 animals in each group and analyzed for transaminase levels. Two hamsters in the AdV6 + Vehicle group had high transaminase levels, while none of the filociclovir- or CDV-treated animals had elevated serum transaminase levels (Fig. 7; alanine aminotransferase [ALT] shown).

### Virus burden in the liver

At 5 days post challenge, untreated AdV6-infected hamsters had high virus burden in their liver (Fig. 8). Treatment with 30mg/kg filociclovir suppressed AdV6 replication to a nondetectable level (Fig. 8). There was one animal in the AdV6 + 10mg/kg filociclovir group with a very low liver virus burden, while we could not detect AdV6 in the liver of any of the other animals in this group (Fig. 8). CDV treatment reduced virus replication to very low to nonquantifiable levels (Fig. 8).

### Conclusions

Data with the two lower doses of filociclovir (10mg/kg and 30mg/kg) are favorable. Treatment with either of these two doses inhibited virus replication (Fig. 8), mitigated liver damage (Fig. 7), and prevented morbidity and mortality (Fig. 6). The 10mg/kg filociclovir dose is slightly less efficacious than the 30mg/kg dose (see marginally lower body weight gain in Fig. 6 and marginally higher virus burden in the liver in Fig. 8). The 60mg/kg and 100mg/kg filociclovir doses are toxic for AdV6-infected hamsters; these two groups and the Vehicle + 100mg/kg group were removed from the study.

### Example 6. Determining the Therapeutic Efficacy of Low Dose Filociclovir Against Intravenously Administered Human AdV6 in Immunosuppressed Male Syrian Hamsters

Next, we assessed whether low doses of filociclovir exhibit anti-adenoviral efficacy in immunosuppressed Syrian hamsters infected intravenously (i.v.) with AdV6. Three dose levels were tested: 1, 3, and 10mg/kg p.o. q.d.

All hamsters were immunosuppressed using cyclophosphamide (CP) administered intraperitoneally at a dose of 140mg/kg, and then twice weekly at a dose of 100mg/kg. Filociclovir in powdered form was suspended in 0.4% carboxymethyl cellulose (Sigma C5678) at 0.1, 0.3, and 1.0mg/ml and sonicated to visual homogeneity. Filociclovir was made up once weekly, aliquoted into daily portions, and stored at 4°C. Aliquots were allowed to equilibrate to room temperature before dosing.

Hamsters were distributed into 7 groups, 15 hamsters/group (see Table 4), immunosuppressed as described above, and injected i.v. with vehicle (Groups 1-2) or 4x10¹⁰ PFU/kg of AdV6 (Groups 3-7). Groups 1 and 3 received drug vehicle (p.o. q.d.), Groups 2 and 6 received 10mg/kg filociclovir (p.o. q.d.), Group 4 received 3mg/kg filociclovir (p.o. q.d.), Group 5 received 1mg/kg filociclovir (p.o. q.d.), and Group 7 received cidofovir (one dose of 37mg/kg, followed by 20mg/kg doses 3 times weekly). For all groups, drug administration started 1 day before challenge, and continued according to the schedule above for the duration of the study.

**Table 4 - Prophylactic dose escalation study for filociclovir - AdV6-infected male animals**

| **Group #** | **Groups** | **n** | **Work Performed** | | |
|---|---|---|---|---|---|
| | | | **Daily** | **At Necropsy** | |
| | | | | **Day 5** | **Day 14** |
| **1** | **Vehicle** | 5+10 | | Serum ALT, bank liver for histopathology | Bank serum for ALT, liver for histopathology |
| **2** | **Vehicle + filociclovir 10mg/kg p.o. q.d.** | 5+10 | | | |
| **3** | **AdV6 + Vehicle** | 5+10 | | Serum ALT, liver TCID50, bank liver for histopathology | Bank serum for ALT, liver for TCID50, liver for histopathology |
| **4** | **AdV6 + filociclovir 1mg/kg p.o. q.d.** | 5+10 | Dose drug/drug vehicle daily, Observation, Body Weight | | |
| **5** | **AdV6 + filociclovir 3mg/kg p.o. q.d.** | 5+10 | | | |
| **6** | **AdV6 + filociclovir 10mg/kg p.o. q.d.** | 5+10 | | | |
| **7** | **AdV6 + CDV 20mg/kg i.p. 3x weekly** | 5+10 | | | |

The body weights and any signs of morbidity of the animals were monitored and recorded daily. At 5 days post challenge, 5 hamsters (designated at the start of the experiment) from each group were sacrificed, and gross pathological observation was performed. Serum and liver were collected and virus burden in liver was determined by TCID₅₀ assay and the serum was analyzed for transaminase levels. The remaining 10 hamsters were sacrificed at 14 days post challenge. Hamsters that became moribund before Day 14 were sacrificed as needed. In addition to the animals judged moribund by observation, we sacrificed all hamsters that lost more than 20% of their original body weight. For all animals, liver tissue was preserved in formalin for histopathological examination.

### Results

### In-life observations

There were 14 treatment-related deaths in this experiment; 9 in the AdV6 + Vehicle group and 5 in the AdV6 + 1mg/kg filociclovir-treated group (Fig. 9A). Initially, all AdV6-infected hamsters lost body weight, however this was completely reversed by treatment with 10mg/kg filociclovir and partially reversed by treatment with 3mg/kg filociclovir. 1mg/kg filociclovir was unable to mitigate weight loss (Fig. 9B). Cidofovir (CDV) treatment completely reversed body weight loss (Fig. 9B). Filociclovir treatment of uninfected hamsters at 10mg/kg did not result in body weight loss (Fig. 9A).

### Necropsy

At the Day 5 sacrifice, 4 hamsters in the AdV6 + Vehicle and 3 hamsters in the AdV6 + 1 mg/kg filociclovir groups had mottled liver and enlarged gall bladder (a sign of adenovirus pathology). Similar findings were noted in animals that were sacrificed as moribund. No other significant findings were noted at the Day 14 sacrifice.

### Serum chemistry

The 10mg/kg filociclovir q.d. dose prevented AdV6-induced liver damage as determined by serum transaminase levels, while it was largely ineffective at the 1mg/kg filociclovir q.d. level (Fig. 10, ALT shown). Treatment with 3mg/kg filociclovir had a moderate yet significant effect (p=0.0419) (Fig. 10). CDV completely prevented AdV6-induced liver pathology (Fig. 10).

### Virus burden in the liver

The 10mg/kg filociclovir q.d. dose inhibited AdV6 replication in the liver, while it was ineffective at the 1mg/kg q.d. dose (Fig. 11). The 3mg/kg filociclovir q.d. p.o. dose suppressed virus replication to an intermediate level. CDV completely prevented AdV6 replication in the liver (Fig. 11).

### Conclusions

The data suggest that the 10mg/kg p.o. q.d. dose of filociclovir is efficacious in preventing the pathology caused by intravenous infection of immunosuppressed hamsters with AdV6 while 3mg/kg filociclovir had a moderate effect, however 1mg/kg filociclovir was not efficacious.

### Example 7. Determining the Therapeutic Efficacy of Delayed Treatment with 10mg/kg Filociclovir Following Challenge by AdV in Immunosuppressed Male Syrian Hamsters

### Experimental design

Male Syrian hamsters (Envigo, Hackensack, NJ) at 60 to 80g were immunosuppressed by administration of cyclophosphamide (CP) intraperitoneally at a dose of 140mg/kg, and then twice weekly at a dose of 100mg/kg.

Filociclovir in powdered form was suspended in 0.4% carboxymethyl cellulose (Sigma C5678) at 1mg/ml and sonicated to visual homogeneity. Filociclovir was made up once weekly, aliquoted into daily portions, and stored at 4°C. Aliquots are allowed to equilibrate to room temperature before dosing.

Hamsters were distributed into 8 groups, 15 hamsters each (Table 5), immunosuppressed as described above, and then injected i.v. with vehicle or 4x10¹⁰ plaque forming units (PFU) of AdV6 per kg. Filociclovir was administered p.o. at 10mg/kg q.d., starting at one day before, or 1, 2, 3, or 4 days after AdV6 injection. An AdV6-infected group that did not receive filociclovir, and groups that received virus vehicle and drug vehicle only or virus vehicle plus drug (started at one day before challenge) were used as controls.

**Table 5 - Therapeutic window study for Filociclovir**

| **Groups** | **n** | **Work Performed** | | |
|---|---|---|---|---|
| | | **Day 7** | **Day 14** | **Daily** |
| **Vehicle** | 5+10 | Sacrifice 5 animals; Gross pathology, Serum transaminase levels | Sacrifice 10 animals; Gross pathology (optional: Serum transaminase levels) | |
| **Vehicle + Drug** | 5+10 | | | |
| **AdV6 + Vehicle** | 5+10 | Sacrifice 5 animals; Gross pathology, Serum transaminase levels, Ad titer in liver | Sacrifice 10 animals; Gross pathology (optional: Serum transaminase levels and Ad titer in liver) | |
| **AdV6 + Drug Day -1** | 5+10 | | | Dose drug/drug vehicle, Observation, Body Weight |
| **AdV6 + Drug Day +1** | 5+10 | | | |
| **AdV6 + Drug Day +2** | 5+10 | | | |
| **AdV6 + Drug Day +3** | 5+10 | | | |
| **AdV6 + Drug Day +4** | 5+10 | | | |

Body weights and any signs of morbidity in the hamsters were recorded daily. At 7 days post challenge, 5 hamsters from each group (designated at the start of the experiment) were sacrificed, and gross pathological observations were performed. Serum and liver were collected and virus burden in liver was determined by TCID₅₀ assay and the serum was analyzed for transaminase levels. The remaining 10 hamsters were sacrificed at 14 days post challenge. Hamsters that became moribund before Day 14 were sacrificed as needed. In addition to the hamsters that were determined to be moribund by observation, we also sacrificed all hamsters that lost more than 20% of their original body weight.

### Results

### In-life observations

There were 14 treatment-related deaths in this experiment; 9 in the AdV6 + Vehicle group and 5 in the AdV6 + 1mg/kg filociclovir group (Fig. 12A). Initially, all AdV6-infected hamsters lost body weight, however, this was completely reversed by treatment with 10mg/kg filociclovir, and partially reversed by treatment with 3mg/kg filociclovir. However, 1mg/kg filociclovir was unable to mitigate weight loss (Fig. 12B). CDV treatment completely reversed body weight loss (Fig. 12B). Treatment of uninfected animals with 10mg/kg filociclovir did not result in body weight loss (Fig. 12B).

### Necropsy

At the Day 5 sacrifice, 4 hamsters in the AdV6 + Vehicle and 3 in the AdV6 + 1mg/kg filociclovir groups had mottled liver and enlarged gall bladder (a sign of adenovirus pathology). Similar findings were noted in hamsters that were sacrificed as moribund. No other significant findings were noted. No significant findings were noted at the Day 14 sacrifice.

### Serum chemistry

The 10mg/kg q.d. filociclovir dose prevented AdV6-induced liver damage as determined by serum transaminase levels, however, filociclovir was largely ineffective at the 1mg/kg q.d. dose level (Fig. 13, ALT shown). Treatment with 3mg/kg filociclovir had a moderate yet significant effect (p=0.0419) (Fig. 13). CDV completely prevented AdV6-induced liver pathology (Fig. 13).

### Virus burden in the liver

The 10mg/kg q.d. filociclovir dose inhibited AdV6 replication in the liver, while it was ineffective at 1mg/kg q.d. (Fig. 14). The 3mg/kg q.d. p.o. filociclovir dose suppressed virus replication to an intermediate level. CDV completely prevented AdV6 replication in the liver (Fig. 14).

### Conclusions

The data demonstrate that the 10mg/kg p.o. q.d. dose of filociclovir is safe and effective in preventing the pathology caused by intravenous infection of immunosuppressed hamsters with AdV6 up to 4 days post-infection. The 1mg/kg filociclovir dose was not efficacious, while the 3mg/kg filociclovir dose had a moderate effect.

Consideration of the foregoing data showed that filociclovir was an effective antiviral agent for inhibiting a range of adenovirus types, leading to the conclusion that filociclovir is useful as a pan-adenoviral therapeutic.

## Claims

1. Filociclovir or a pharmaceutically acceptable salt thereof for use in a method of treating adenovirus infection in a mammal.

2. Filociclovir or a pharmaceutically acceptable salt thereof for use according to Claim 1, wherein said adenovirus is adenovirus 3 (AdV3).

3. Filociclovir or a pharmaceutically acceptable salt thereof for use according to Claim 1, wherein said adenovirus is adenovirus 4 (AdV4).

4. Filociclovir or a pharmaceutically acceptable salt thereof for use according to Claim 1, wherein said adenovirus is adenovirus 5 (AdV5).

5. Filociclovir or a pharmaceutically acceptable salt thereof for use according to Claim 1, wherein said adenovirus is adenovirus 6 (AdV6).

6. Filociclovir or a pharmaceutically acceptable salt thereof for use according to Claim 1, wherein said adenovirus is adenovirus 7 (AdV7).

7. Filociclovir or a pharmaceutically acceptable salt thereof for use according to Claim 1, wherein said adenovirus is adenovirus 8 (AdV8).

8. Filociclovir or a pharmaceutically acceptable salt thereof for use according to Claim 1, wherein said adenovirus is adenovirus 19 (AdV19).

9. Filociclovir or a pharmaceutically acceptable salt thereof for use according to Claim 1, wherein said adenovirus is adenovirus 37 (AdV37).

10. Filociclovir or a pharmaceutically acceptable salt thereof for use according to Claim 1, wherein the mammal is a human.

## Patentansprüche

1. Filociclovir oder ein pharmazeutisch verträgliches Salz davon zur Verwendung in einem Verfahren der Behandlung von Adenovirus-Infektion bei einem Säugetier.

2. Filociclovir oder ein pharmazeutisch verträgliches Salz davon zur Verwendung gemäß Anspruch 1, worin das genannte Adenovirus Adenovirus 3 (AdV3) ist.

3. Filociclovir oder ein pharmazeutisch verträgliches Salz davon zur Verwendung gemäß Anspruch 1, worin das genannte Adenovirus Adenovirus 4 (AdV4) ist.

4. Filociclovir oder ein pharmazeutisch verträgliches Salz davon zur Verwendung gemäß Anspruch 1, worin das genannte Adenovirus Adenovirus 5 (AdV5) ist.

5. Filociclovir oder ein pharmazeutisch verträgliches Salz davon zur Verwendung gemäß Anspruch 1, worin das genannte Adenovirus Adenovirus 6 (AdV6) ist.

6. Filociclovir oder ein pharmazeutisch verträgliches Salz davon zur Verwendung gemäß Anspruch 1, worin das genannte Adenovirus Adenovirus 7 (AdV7) ist.

7. Filociclovir oder ein pharmazeutisch verträgliches Salz davon zur Verwendung gemäß Anspruch 1, worin das genannte Adenovirus Adenovirus 8 (AdV8) ist.

8. Filociclovir oder ein pharmazeutisch verträgliches Salz davon zur Verwendung gemäß Anspruch 1, worin das genannte Adenovirus Adenovirus 19 (AdV19) ist.

9. Filociclovir oder ein pharmazeutisch verträgliches Salz davon zur Verwendung gemäß Anspruch 1, worin das genannte Adenovirus Adenovirus 37 (AdV37) ist.

10. Filociclovir oder ein pharmazeutisch verträgliches Salz davon zur Verwendung gemäß Anspruch 1, worin das Säugetier ein Mensch ist.

## Revendications

1. Filociclovir, ou un sel pharmaceutiquement acceptable de celui-ci, destiné à être utilisé dans un procédé de traitement d'une infection à adénovirus chez un mammifère.

2. Filociclovir, ou un sel pharmaceutiquement acceptable de celui-ci, destiné à être utilisé selon la revendication 1, dans lequel ledit adénovirus est l'adénovirus 3 (AdV3).

3. Filociclovir, ou un sel pharmaceutiquement acceptable de celui-ci, destiné à être utilisé selon la revendication 1, dans lequel ledit adénovirus est l'adénovirus 4 (AdV4).

4. Filociclovir, ou un sel pharmaceutiquement acceptable de celui-ci, destiné à être utilisé selon la revendication 1, dans lequel ledit adénovirus est l'adénovirus 5 (AdV5).

5. Filociclovir, ou un sel pharmaceutiquement acceptable de celui-ci, destiné à être utilisé selon la revendication 1, dans lequel ledit adénovirus est l'adénovirus 6 (AdV6).

6. Filociclovir, ou un sel pharmaceutiquement acceptable de celui-ci, destiné à être utilisé selon la revendication 1, dans lequel ledit adénovirus est l'adénovirus 7 (AdV7).

7. Filociclovir, ou un sel pharmaceutiquement acceptable de celui-ci, destiné à être utilisé selon la revendication 1, dans lequel ledit adénovirus est l'adénovirus 8 (AdV8).

8. Filociclovir, ou un sel pharmaceutiquement acceptable de celui-ci, destiné à être utilisé selon la revendication 1, dans lequel ledit adénovirus est l'adénovirus 19 (AdV19).

9. Filociclovir, ou un sel pharmaceutiquement acceptable de celui-ci, destiné à être utilisé selon la revendication 1, dans lequel ledit adénovirus est l'adénovirus 37 (AdV37).

10. Filociclovir, ou un sel pharmaceutiquement acceptable de celui-ci, destiné à être utilisé selon la revendication 1, dans lequel le mammifère est un être humain.
